(19) Europäisches Patentamt
European Patent Office
Office européen des brevets

(11) Veröffentlichungsnummer: **0 173 190 B1**

# (12) EUROPÄISCHE PATENTSCHRIFT

(45) Veröffentlichungstag der Patentschrift: **11.09.91**

(51) Int. Cl.$^5$: **C07D 239/26**

(21) Anmeldenummer: **85110312.7**

(22) Anmeldetag: **17.08.85**

## (54) Verfahren zur Herstellung von 5-Acylpyrimidinen.

(30) Priorität: **29.08.84 DE 3431689**

(43) Veröffentlichungstag der Anmeldung:
**05.03.86 Patentblatt 86/10**

(45) Bekanntmachung des Hinweises auf die Patenterteilung:
**11.09.91 Patentblatt 91/37**

(84) Benannte Vertragsstaaten:
**CH DE FR GB IT LI**

(56) Entgegenhaltungen:

**ACTA CHEMICA SCANDINAVICA, Series B, Organic chemistry and biochemistry, Band B36, Nr. 8, 1982, Seiten 529-531, Chemical Societies in Denmark, Finland, Norway and Sweden; T. BENNECHE et al.: "Enols of 1,1-diformylacetone in the Synthesis of 5-acylpyrimidines"**

**Formation of C-C Bonds, G. Thieme, Stuttgart, 1973, Vol. I, pp. 229-331**

**Collect. Czech. Comm. 28, S. 869-881**

(73) Patentinhaber: **BAYER AG**

**W-5090 Leverkusen 1 Bayerwerk(DE)**

(72) Erfinder: **Elbe, Hans-Ludwig, Dr.**
**Dasnöckel 59**
**W-5600 Wuppertal 11(DE)**
Erfinder: **Homwood, Graham, Dr.**
**Krutscheider Weg 105**
**W-5600 Wuppertal 11(DE)**

**EP 0 173 190 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von 5-Acylpyrimidinen, welche als Zwischenprodukte zur Herstellung von Stoffen mit pflanzenwachstumsregulierender und fungizider Wirksamkeit eingesetzt werden können.

Es ist bereits bekannt, daß sich Pyrimidinylcarbinole als Zwischenprodukte zur Herstellung von Benzyl-pyrimidinyl-alkyl-ethern, mit pflanzenwuchsregulierenden und fungiziden Eigenschaften verwenden lassen (DE-A-3 105 374).

So erhält man Benzyl-pyrimidinyl-alkyl-ether, wenn man Pyrimidinylcarbinole mit Benzylhalogeniden nach folgendem Reaktionsschema umsetzt:

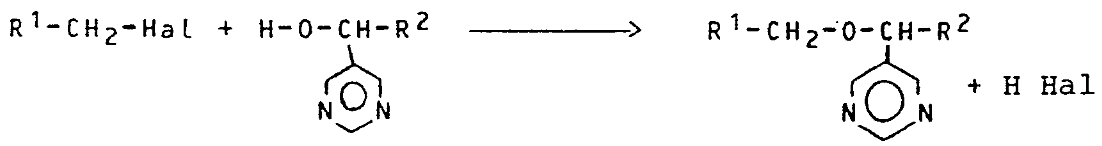

$R^1$ = gegebenenfalls substituiertes Phenyl,
$R^2$ = Alkyl, gegebenenfalls substituiertes Cycloalkyl oder gegebenenfalls substituiertes Phenyl,
Hal = Halogen.

Nachteilig an diesem bekannten Verfahren ist jedoch, daß die als Ausgangsstoffe benötigten Pyrimidinylcarbinole nur schwierig zugänglich sind. So lassen sich diese Pyrimidinylcarbinole dadurch herstellen, daß man Pyrimidinylhalogenide mit einer alkalimetallorganischen Verbindung, wie z.B. n-Butyllithium und anschließend mit entsprechenden Aldehyden bei tiefen Temperaturen, zum Beispiel zwischen -100 und -120 °C, umsetzt (vgl. DE-A-3 105 374). Das Arbeiten mit metallorganischen Verbindungen bei derartig tiefen Temperaturen macht eine rationelle Anwendung dieses Verfahrens im technischen Maßstab nahezu unmöglich.

Ein weiterer Nachteil dieses Verfahren besteht darin, daß der Preis für das als Ausgangsstoff benötigte Pyrimidinylhalogenid extrem hoch ist. Es besteht deshalb ein großes Interesse an einem Verfahren, das eine einfachere Synthese der sehr gut wirksamen Benzyl-pyrimidinyl-alkyl-ether der oben angegebenenen allgemeinen Formel ermöglicht.

Außerdem ist bekannt, daß man 5-Acetylpyrimidin ausgehend von 5-Acetyl-uracil über 2,4-Dichlor-5-acetylpyrimidin durch reduktive Enthalogenierung erhalten kann (vgl. Arch. Pharm. 299, 362 (1966)). Die Ausbeute an Endprodukt ist bei diesem mehrstufigen Prozeß allerdings sehr niedrig.

Ferner ist bekannt, daß man 5-(2-Hydroxybenzoyl)-pyrimidin erhält, wenn man Chromon-3-carbaldehyd mit Formamidin umsetzt (vgl. Synthesis 1976, 274). Auch hier ist die Ausbeute nicht zufriedenstellend. Außerdem ist diese Verfahrensvariante nicht auf im Phenylteil beliebig substituierte 5-Benzoyl-pyrimidine verallgemeinerungsfähig.

Weiterhin ist bekannt, daß bestimmte substituierte 5-Acylpyrimidine erhalten werden können, wenn man ein Methylketon formyliert, weiter zum Enol-Acetat umsetzt, das Enol-Acetat mit Ethylorthoameisensäureester formyliert und schließlich den Ringschluß zum Pyrimidin mit beispielsweise Methylisothioharnstoff vornimmt (vgl. Acta Chemica. Scandinavia B 36, 529-31 (1982)). Dieses Verfahren hat den Nachteil einer Mehrstufenreaktion, wobei die Zwischenstufen isoliert werden.

Es wurde nun gefunden, daß sich 5-Acylpyrimidine der Formel

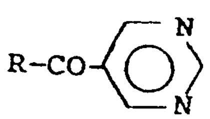 (I)

in welcher

R für Isopropyl, tert.-Butyl, n-Pentyl, Neopentyl, n-Hexyl, n-Heptyl, n-Octyl, geradkettiges oder verzweigtes Alkenyl mit 3 bis 6 Kohlenstoffatomen, geradkettiges oder verzweigtes Alkinyl mit 3 bis 6 Kohlenstoffatomen, für jeweils gegebenenfalls einfach bis dreifach, gleich oder verschieden durch Alkyl mit 1 bis 4 Kohlenstoffatomen substituiertes Cycloalkyl und Cycloalkylalkyl mit jeweils 3 bis 7 Kohlenstoffatomen im Cycloalkylteil und 1 bis 4 Kohlenstoffatomen im Alkylteil; für gegebenenfalls einfach bis dreifach, gleich oder verschieden substituiertes Phenyl, wobei als Substituenten genannt seien: Halogen, Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 5 gleichen oder verschiedenen Halogenatomen, wie Fluor- und Chloratomen; Nitro, Cyano, Alkoxycarbonyl mit 1 bis 4 Kohlenstoffatomen im Alkoxyteil; sowie jeweils gegebenenfalls durch Halogen substituiertes Phenyl und Phenoxy; ferner für gegebenenfalls einfach bis dreifach, gleich oder verschieden substituiertes 5- bis 6-gliedriges Heteroaryl mit 1 bis 3 Sauerstoff-, Stickstoff- und/oder Schwefelatomen, wobei als Substituenten die obengenannten Phenylsubstituenten infrage kommen, sowie für die Gruppierungen

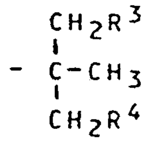 , 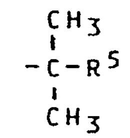 und 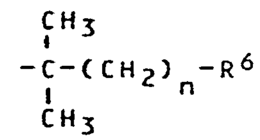 ,

wobei

- $R^3$ für Fluor, Chlor oder Brom steht;
- $R^4$ für Wasserstoff, Fluor, Chlor oder Brom steht;
- $R^5$ für geradkettiges oder verzweigtes Alkenyl mit 2 bis 4 Kohlenstoffatomen, geradkettiges oder verzweigtes Alkinyl mit 3 bis 5 Kohlenstoffatomen, sowie für die CHO-Gruppe und Alkoximinomethyl mit 1 bis 4 Kohlenstoffatomen in jedem Alkoxyteil, Dialkoxymethyl mit 1 bis 4 Kohlenstoffatomen in jedem Alkoxyteil sowie gegebenenfalls substituierte Dixolane und Dioxane steht,
- $R^6$ für Cyano, für gegebenenfalls einfach bis dreifach, gleich oder verschieden substituiertes Phenyl steht, wobei als Substituenten die oben bereits genannten Phenylsubstituenten infrage kommen, sowie für die Gruppierungen $-XR^7$ und $-CONR^8R^9$ steht, wobei
- $R^7$ für geradkettiges oder verzweigtes Alkyl mit 1 bis 6 Kohlenstoffatomen, Halogenalkyl mit 1 bis 4 Kohlenstoffatomen und 1 bis 5 gleichen oder verschiedenen Halogenatomen, wie Fluor- und Chloratomen, sowie für jeweils gegebenenfalls einfach bis dreifach, gleich oder verschieden substituiertes Phenyl oder Phenylalkyl mit 1 bis 2 Kohlenstoffatomen im Alkylteil steht, wobei jeweils als Substituenten die oben bereits genannten Phenylsubstituenten infrage kommen;
- $R^8$ für Wasserstoff, geradkettiges oder verzweigtes Alkyl mit 1 bis 4 Kohlenstoffatomen oder für gegebenenfalls einfach bis dreifach, gleich oder verschieden substituiertes Phenyl steht, wobei als Substituenten die oben bereits genannten Phenylsubstituenten infrage kommen;
- $R^9$ für Wasserstoff oder geradkettiges oder verzweigtes Alkyl mit 1 bis 4 Kohlenstoffatomen steht;
- X für Sauerstoff oder Schwefel steht und
- n für die Zahlen 0, 1 oder 2 steht;

herstellen lassen, indem man Methylketone der Formel

$R-CO-CH_3$ (II)

in welcher

R die oben angegebene Bedeutung hat,

a) in einer 1. Stufe mit einem Ameisensäurealkylester mit 1 bis 4 Kohlenstoffatomen in der Alkylgruppe oder Ameisensäure-phenylester in Gegenwart von Alkalialkoholaten und gegebenenfalls in Gegenwart eines Verdünnungsmittels umsetzt und gegebenenfalls ansäuert; danach in einer 2. Stufe die entstehenden Enol-ketone der Formel

$R-CO-CH=CH-OR^{10}$ (III)

in welcher

R die oben angegebene Bedeutung hat und

$R^{10}$ für Wasserstoff oder einen Baserest steht,

mit oder ohne Zwischenisolierung mit Formamidin, wobei das Formamidin gegebenenfalls in Form eines Formamidinsalzes vorliegen kann, gegebenenfalls in Gegenwart eines Verdünnungsmittels, umsetzt; anschließend in einer 3. Stufe die entstehenden Formamidin-Derivate der Formel

$R-CO-CH_2-CH=N-CH=NH$ (IV)

in welcher
R die oben angegebene Bedeutung hat,
mit oder ohne Zwischenisolierung mit einen
α) Formamid-Acetale der Formel

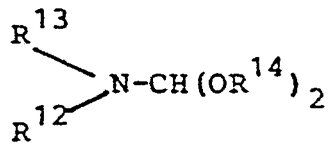

(VII)

in welcher
$R^{12}$ und $R^{13}$ unabhängig voneinander für Alkyl mit 1 bis 4 Kohlenstoffatomen stehen und
$R^{14}$ für Alkyl mit 1 bis 4 Kohlenstoffatomen steht,
oder
β) Aminalester der Formel

$R^{14}O\text{-}CH(NR^{12}R^{13})_2$ (VIII)

in welcher
$R^{12}$, $R^{13}$ und $R^{14}$ die oben angegebene Bedeutung haben;
oder
γ) Formiminium-Halogenide der Formel

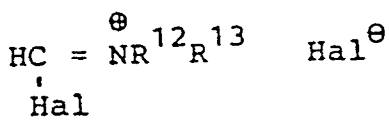

(IX)

in welcher
$R^{12}$ und $R^{13}$ die oben angegebene Bedeutung haben und
Hal für Halogen steht,
gegebenenfalls in Gegenwart eines Verdünnungsmittels, bei Temperaturen zwischen -10 und +10 °C umsetzt;
und in einer 4. Stufe die entstehenden Verbindungen der Formel

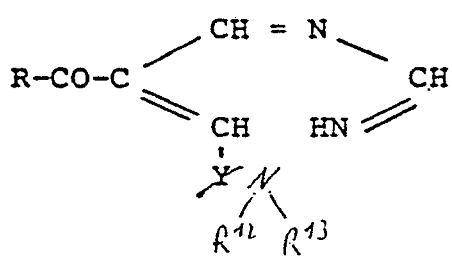

(V)

in welcher
R die oben angegebene Bedeutung hat
mit oder ohne Zwischenisolierung thermisch cyclisiert.

Schließlich wurde gefunden, daß sich die 5-Acyl-pyrimidine der Formel (I) sehr gut als Zwischenprodukte zur Herstellung von Benzyl-pyrimidinyl-alkyl-ethern mit pflanzenwuchsregulierender und fungizider Wirksamkeit eignen.

Der Verlauf des erfindungsgemäßen Verfahrens ist als äußerst überraschend zu bezeichnen.

So war im Hinblick auf den bekannten Stand der Technik zu erwarten, daß das gebildete Zwischenprodukt der Formel (IV) (Herstellung aus dem formalen Diketon $R\text{-}CO\text{-}CH_2\text{-}CHO$ und Formamidin) nach folgendem Reaktionsschema zum entsprechenden 4-substituierten Pyrimidin cyclisiert:

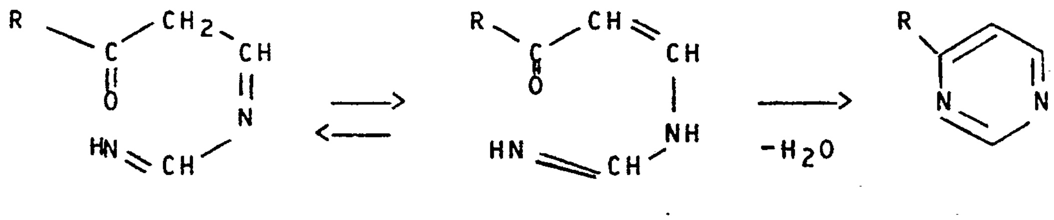


(IV) (IVa)

Derartige Cyclisierungen von β-Diketonen mit Amidinen zu 4-substituierten Pyrimidinen sind in der Literatur häufig beschrieben worden (vgl. z.B. D.J. Brown, The Pyrimidines, S. 36, Interscience Publishers, 1962). Überraschenderweise tritt eine solche Ringschlußreaktion im Verlauf des erfindungsgemäßen Verfahren jedoch nicht auf.

Das erfindungsgemäße Verfahren zeichnet sich durch eine Reihe von Vorteilen aus. So lassen sich die gewünschten 5-Acylpyrimidine der Formel (I), - auch in technischem Maßstab -, in einfacher Art und Weise herstellen. Die Ausbeuten liegen relativ hoch. Besonders günstig ist, daß man keine der auftretenden Zwischenstufen isolieren muß, so daß es sich im Prinzip um ein Eintopfverfahren handelt, wodurch sich besonders gute Raum-Zeit-Ausbeuten erzielen lassen. Im übrigen ist hervorzuheben, daß die nach dem erfindungsgemäßen Verfahren zugänglichen 5-Acylpyrimidine der Formel (I) durch anschließende Reduktion und Umsetzung der dabei entstehenden Carbinole mit Benzylhalogeniden in Benzyl-pyrimidinyl-alkyl-ether überführt werden können, welche pflanzenwuchsregulierende und fungizide Eigenschaften besitzen. Dadurch steht jetzt eine Methode zur Synthese der letztgenannten Wirkstoffe zur Verfügung, die wesentlich einfacher ist als das bisher bekannte Verfahren. Außerdem ist dieses neue Verfahren im Gegensatz zu dem bisherigen Verfahren auch ohne Schwierigkeiten in technischem Maßstab anwendbar.

Die 5-Acylpyrimidineder Formel

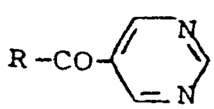 (I)

in welcher

R die oben angegebene Bedeutung hat

sind neu.

Besonders bevorzugt sind diejenigen Verbindungen der Formel (I), in denen

R für Isopropyl, tert.-Butyl, Neopentyl, Allyl, Propargyl, für gegebenenfalls einfach oder zweifach, gleich oder verschieden durch Methyl, Ethyl, Isopropyl oder tert.-Butyl substituiertes Cyclopropyl, Cyclobutyl, Cyclopentyl oder Cyclohexyl, für gegebenenfalls einfach oder zweifach, gleich oder verschieden substituiertes Phenyl steht, wobei als Substituenten genannt seien:
Fluor, Chlor, Methyl, Ethyl, Isopropyl, tert.-Butyl, Methoxy, Methylthio, Trifluormethyl, Trifluormethoxy, Trifluormethylthio, Nitro, Cyano, Methoxycarbonyl sowie jeweils gegebenenfalls durch Chlor oder Fluor substituiertes Phenyl und Phenoxy; weiterhin für jeweils gegebenenfalls einfach bis zweifach, gleich oder verschieden substituiertes Furyl, Thienyl, Benzofuryl, Benzothienyl, Pyridinyl oder Pyrimidinyl steht, wobei als Substituenten die oben genannten Phenylsubstituenten infrage kommen; sowie für die Gruppierungen

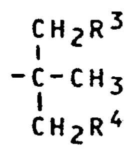 , 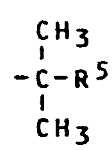 und 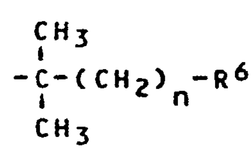

steht, wobei

$R^3$ für Fluor oder Chlor steht;

$R^4$ für Wasserstoff, Fluor oder Chlor steht;

$R^5$ für Vinyl, Propargyl, die -CH=O-Gruppe, Methoximinomethyl, Dimethoxymethyl, den Dioxolan-

oder 1,3-Dioxan-Rest steht;

$R^6$ für Cyano oder gegebenenfalls einfach oder zweifach, gleich oder verschieden substituiertes Phenyl steht, wobei als Substituenten die oben bereits genannten Phenylsubstituenten infrage kommen, sowie für die Gruppierungen $-XR^7$ und $-CONR^8R^9$ steht; wobei

$R^7$ für geradkettiges oder verzweigtes Alkyl mit 1 bis 4 Kohlenstoffatomen, Halogenalkyl mit 1 bis 2 Kohlenstoffatmomen und 1 bis 5 gleichen oder verschiedenen Halogenatomen, wie Fluor- und Chloratomen, sowie für jeweils gegebenenfalls einfach oder zweifach, gleich oder verschieden substituiertes Phenyl und Benzyl steht, wobei jeweils als Substituenten die oben bereits genannten Phenylsubstituenten infrage kommen;

$R^8$ für Wasserstoff, Methyl, Ethyl, Isopropyl oder für gegebenenfalls einfach oder zweifach, gleich oder verschieden substituiertes Phenyl steht, wobei als Substituenten die oben bereits genannten Phenylsubstituenten infrage kommen;

$R^9$ für Wasserstoff, Methyl oder Isopropyl steht;

X für Sauerstoff oder Schwefel steht und

n für die Zahlen 0, 1 oder 2 steht.

Verwendet man beispielsweise (Methoxy-tert.-butyl)-methylketon als Ausgangsstoff, Ameisensäureethylester in Gegenwart von Natriummethylat als Formylierungsreagenz, Formamidin in Form des Acetats und N,N-Dimethylformamid-dimethylacetal als Aminoformylierungsmittel, so kann der Reaktionsablauf des erfindungsgemäßen Verfahrens durch das folgende Formelschema wiedergegeben werden:

1.Stufe

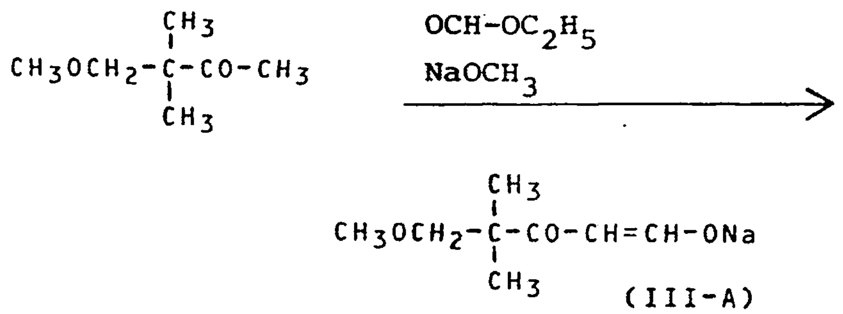


(III-A)

(ohne Isolierung)

2.Stufe

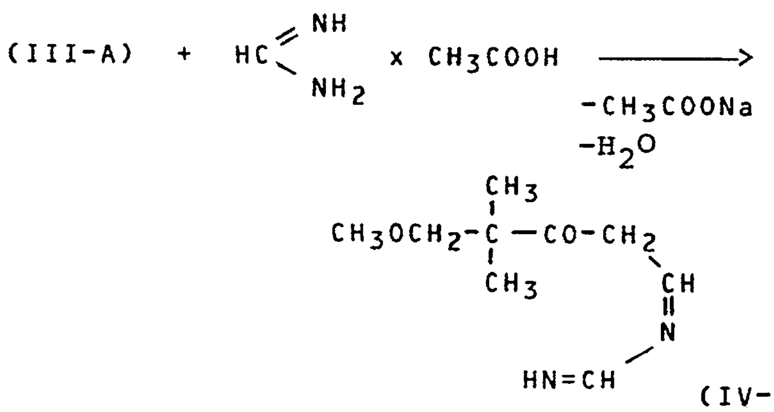


(IV-A)

(ohne Isolierung)

3.Stufe

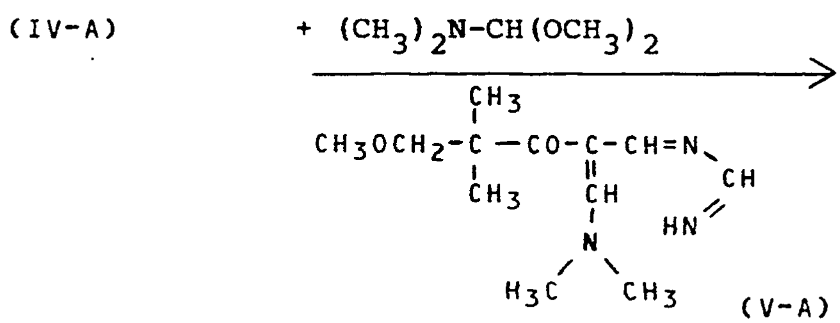


(V-A)

(ohne Isolierung)

4. Stufe

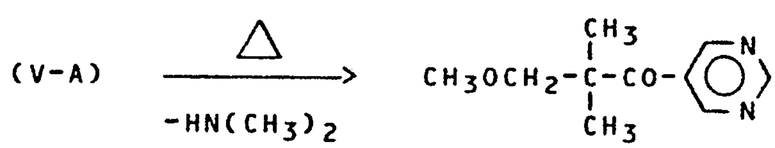

Die bei der Durchführung des erfindungsgemäßen Verfahrens als Ausgangsstoffe benötigten Methylketone sind durch die Formel (II) allgemein definiert. In dieser Formel steht

R vorzugsweise für Isopropyl, tert.-Butyl, n-Pentyl, Neopentyl, n-Hexyl, n-Heptyl, n-Octyl, Allyl, Propargyl, für gegebenenfalls einfach oder zweifach, gleichartig oder verschieden durch Methyl, Ethyl, Isopropyl oder tert.-Butyl substituiertes Cyclopropyl, Cyclobutyl, Cyclopentyl oder Cyclohexyl, für gegebenenfalls einfach oder zweifach, gleich oder verschieden substituiertes Phenyl, wobei als Substituenten genannt seien:
Fluor, Chlor, Hydroxy, Methyl, Ethyl, Isopropyl, tert.-Butyl, Methoxy, Methylthio, Trifluormethyl, Trifluormethoxy, Trifluormethylthio, Nitro, Cyano, Methoxycarbonyl sowie jeweils gegebenenfalls durch Chlor oder Fluor substituiertes Phenyl und Phenoxy; weiterhin für jeweils gegebenenfalls einfach bis zweifach, gleich oder verschieden substituiertes Furyl, Thienyl, Benzofuryl, Benzothienyl, Pyridinyl oder Pyrimidinyl steht, wobei als Substituenten die oben genannten Phenylsubstituenten infrage kommen; sowie für die Gruppierungen

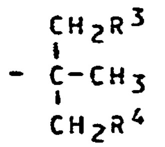 , 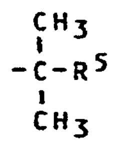 und 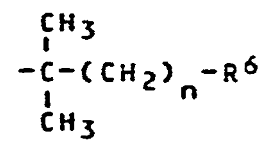 ,

steht, wobei

$R^3$ für Fluor oder Chlor;

$R^4$ für Wasserstoff, Fluor oder Chlor;

$R^5$ für Vinyl, Propargyl, die -CH=O-Gruppe, Methoximinomethyl, Dimethoxymethyl, den Dioxolan- oder 1,3-Dioxan-Rest;

$R^6$ für Cyano oder gegebenenfalls einfach oder zweifach, gleich oder verschieden substituiertes Phenyl steht, wobei als Substituenten die oben bereits genannten Phenylsubstituenten infrage kommen, sowie für die Gruppierungen $-XR^7$ und $-CONR^8R^9$; wobei

$R^7$ für geradkettiges oder verzweigtes Alkyl mit 1 bis 4 Kohlenstoffatomen, Halogenalkyl mit 1 bis 2 Kohlenstoffatmomen und 1 bis 5 gleichen oder verschiedenen Halogenatomen, wie Fluor- und Chloratomen, sowie für jeweils gegebenenfalls einfach oder zweifach, gleich oder verschieden substituiertes Phenyl und Benzyl, wobei jeweils als Substituenten die oben bereits genannten Phenylsubstituenten infrage kommen;

$R^8$ für Wasserstoff, Methyl, Ethyl, Isopropyl oder für gegebenenfalls einfach oder zweifach, gleich oder verschieden substituiertes Phenyl, wobei als Substituenten die oben bereits genannten Phenylsubstituenten infrage kommen;

$R^9$ für Wasserstoff, Methyl oder Isopropyl;

X für Sauerstoff oder Schwefel und

n für die Zahlen 0, 1 oder 2 steht.

Die Methylketone der Formel (II) sind bekannt oder lassen sich in einfacher Weise nach bekannten Verfahren herstellen.

Als Formylierungsmittel kommen für die Durchführung der ersten Stufe Ameisensäurealkylester mit 1 bis 4 Kohlenstoffatomen in der Alkylgruppe oder Ameisensäure-phenylester in Frage. Diese Formylierungsmittel sind allgemein bekannte Verbindungen der organischen Chemie.

Das bei der Durchführung der 2. Stufe als Reaktionskomponente zu verwendende Formamidin kann entweder als solches oder in Form eines Salzes mit einer organischen oder anorganischen Säure eingesetzt werden. Als Säuren seien beispielsweise die Chlorwasserstoffsäure oder die Essigsäure genannt.

Als Aminoformylierungsmittel kommen bei der Durchführung der 3. Stufe in Frage:

α) Formamid-Acetale der Formel

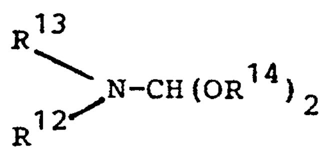 **(VII)**

in welcher

$R^{12}$ und $R^{13}$ unabhängig voneinander für Alkyl mit 1 bis 4 Kohlenstoffatomen stehen und

$R^{14}$ für Alkyl mit 1 bis 4 Kohlenstoffatomen steht,

β) Aminalester der Formel

$$R^{14}\text{-O-CH}(NR^{12}R^{13})_2 \qquad (VIII)$$

in welcher

$R^{12}$, $R^{13}$ und $R^{14}$ die oben angegebene Bedeutung haben;

und

γ) Formiminium-Halogenide der Formel

$$\underset{\displaystyle Hal}{HC} = \overset{\oplus}{N}R^{12}R^{13} \qquad Hal^{\ominus} \qquad (IX)$$

in welcher

$R^{12}$ und $R^{13}$ die oben angegebene Bedeutung haben und

Hal für Halogen steht.

In den oben genannten Verbindungen der Formeln (VII), (VIII) und (IX) stehen

$R^{12}$ und $R^{13}$ unabhängig voneinander besonders bevorzugt für Methyl, Ethyl, n-Propyl, Isopropyl oder n-Butyl.

In den Verbindungen der Formeln (VII) und (VIII) steht $R^{14}$ besonders bevorzugt für Methyl, Ethyl oder n-Propyl.

In den Verbindungen der Formel (IX) steht Hal besonders bevorzugt für Chlor oder Brom.

Die Aminoformylierungsmittel der Formeln (VII), (VIII) und (IX) sind allgemein bekannte Verbindungen der organischen Chemie.

Als Verdünnungsmittel kommen für alle Stufen vorzugsweise inerte, aprotische Lösungsmittel infrage. Hierzu gehören vorzugsweise Ether, wie Diethylether, Diisopropylether, 1,2-Dimethoxyethan und Tetrahydrofuran; aromatische Kohlenwasserstoffe, wie Benzol, Toluol und Xylol; sowie aliphatische Kohlenwasserstoffe, wie Hexan und Octan.

Die Formylierungsreaktion gemäß der 1. Stufe wird in Gegenwart von Alkalialkoholaten, wie Natriummethylat, Kaliumethylat, Lithiumpropylat und Kalium-tert.-butylat durchgeführt.

Als Säuren, die bei der Durchführung der ersten Stufe zur Acidifizierung des Reaktionsgemisches eingesetzt werden können, kommen alle für derartige Zwecke üblichen Säuren in Betracht. Vorzugsweise verwendbar ist Salzsäure.

In Abhängigkeit von der Base, die bei der Durchführung der ersten Stufe des erfindungsgemäßen Verfahrens verwendet wird, und in Abhängigkeit davon, ob in der ersten Stufe eine Acidifizierung des Reaktionsgemisches vorgenommen wird, variiert in den Verbindungen der Formel (III) die Bedeutung des Substituenten $R^{10}$. Werden Alkalimetallalkoholate als Basen eingesetzt, so resultieren Verbindungen der Formel (III), in denen $R^{10}$ für ein Alkalimetall, wie beispielsweise Lithium, Natrium oder Kalium, steht. Wird das Reaktionsgemisch angesäuert, so bilden sich Enolketone der Formel (III), in denen $R^{10}$ für Wasserstoff steht.

Die Reaktionstemperaturen können bei der Durchführung des erfindungsgemäßen Verfahrens in einem größeren Bereich variiert werden. Im allgemeinen arbeitet man

- bei der 1. Stufe zwischen -25 und +60 °C, vorzugsweise zwischen -10 und +40 °C;
- bei der 2. Stufe zwischen -25 und +50 °C, vorzugsweise zwischen -10 und +10 °C;
- bei der 3. Stufe zwischen -10 und +10 °C;
- bei der thermischen Cylisierung in der letzten Stufe zwischen 0 und +100 °C, vorzugsweise zwischen +50 und +100 °C.

Bei der Durchführung des erfindungsgemäßen Verfahrens setzt man auf 1 Mol Methylketon der Formel (II) vorzugsweise äquimolare Mengen oder einen geringen Überschuß, vorzugsweise bis zu 1,5 Mol, an Formylierungsmittel, Aminoformylierungsmittel beziehungsweise an Formamidin ein. Die Aufarbeitung und Isolierung der Verbindungen der Formel (I) bzw. gegebenenfalls auch der einzelnen Zwischenprodukte der Formeln (III), (IV) und (V) erfolgt nach üblichen Methoden.

Wie schon erwähnt stellen die 5-Acylpyrimidine der Formel (I) interessante Zwischenprodukte zur Synthese von Benzylpyrimidinylalkylethern der Formel

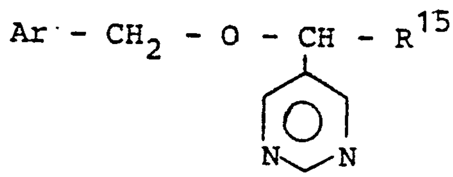

(X)

in welcher

Ar für gegebenenfalls substituiertes Phenyl steht und

$R^{15}$ für Alkyl, gegebenenfalls substituiertes Cycloalkyl oder gegebenenfalls substituiertes Phenyl steht,

dar.

Sie werden erhalten, indem man 5-Acylpyrimidine der Formel

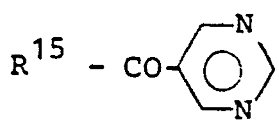

(Ib)

in welcher

$R^{15}$ die oben angegebene Bedeutung hat,

in üblicher Weise reduziert und die so erhaltenen Pyrimidinyl-carbinole der Formel

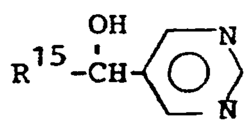

(XI)

in welcher

$R^{15}$ die oben angegebene Bedeutung hat,

mit Benzylhalogeniden der Formel

$Ar - CH_2 - Hal$ (XII)

in welcher

Ar die oben angegebene Bedeutung hat und

Hal für Halogen steht,

in Gegenwart eines Lösungsmittels und gegebenenfalls in Gegenwart einer starken Base umsetzt (vgl. DE-A-3 105 374).

Das erfindungsgemäße Verfahren wird durch die nachfolgenden Beispiele veranschaulicht.

Herstellungsbeispiele

Beispiel 1

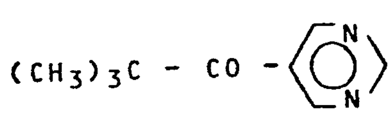

(I-1)

Verfahrensvariante (a) mit Isolierung des Zwischenproduktes der Formel (III-1)

1. Stufe

$(CH_3)_3C-CO-CH=CH-OK$ (III-1)

Zu einer Mischung aus 50,0 g (0,5 Mol) Pinakolon und 42,0 g (0,5 Mol) Ameisensäuremethylester gibt man unter Kühlung bei 15 bis 20° C 56,0 g (0,5 Mol) Kalium-tert.-butylat. Man läßt das Reaktionsgemisch 12 Stunden bei 30° C nachrühren. Zur Aufarbeitung wird mit 400 ml Diethylether versetzt, das ausgefallene Produkt abgesaugt und getrocknet. Man erhält 62,3 g (75 % der Theorie) 4,4-Dimethyl-1-hydroxy-1-penten-3-on als Kaliumsalz in Form eines hellgelben Pulvers.

2. bis 4. Stufe

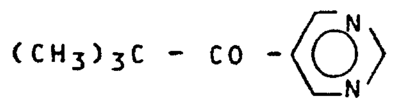

(I-1)

166,2 g (1 Mol) Kaliumsalz des 4,4-Dimethyl-1-hydroxy-1-penten-3-ons (vgl. 1. Stufe) in 800 ml Tetrahydrofuran werden bei 0° C portionsweise mit 132,1 g (1,27 Mol) Formamidinacetat in 800 ml Tetrahydrofuran versetzt. Man läßt das Reaktionsgemisch 15 Minuten bei 0° C nachrühren und versetzt es anschließend mit 165,4 g (1,39 Mol) N,N-Dimethylformamid-dimethylacetal. Man läßt 1 Stunde bei 0° C und 6 Stunden bei 50 bis 55° C nachrühren. Zur Aufarbeitung wird abgekühlt, vom Kaliumacetat abgesaugt und das Filtrat fraktioniert destilliert. Man erhält 49,2 g (30 % der Theorie) tert.-Butyl-5-pyrimidinyl-keton vom Siedepunkt $Kp_{16}$ = 102-105° C.

Beispiel 2

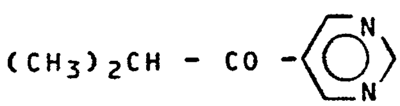

(I-2)

Verfahrensvariante (a) mit Isolierung des Zwischenproduktes der Formel (III-2)

1. Stufe

$(CH_3)_2CH-CO-CH=CH-ONa$ (III-2)

216 g (4 Mol) Natriummethylat in 1,5 l Diethylether werden tropfenweise zunächst bei 25° C mit 344 g (4 Mol) Isopropylmethyl-keton und danach bei 30-35° C mit 296 g (4 Mol) Ameisensäureethylester versetzt. Man läßt das Reaktionsgemisch sechs Stunden bei 25° C nachrühren und saugt dann das ausgefallene Natriumsalz ab. Man erhält 360 g (66 % der Theorie) 1-Hydroxy-4-methyl-1-penten-3-on als Natriumsalz.

2. bis 4. Stufe

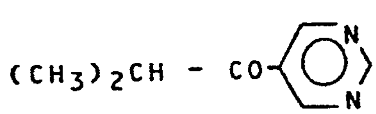

(I-2)

250 g (1,84 Mol) Natriumsalz des 1-Hydroxy-4-methyl-1-penten-3-ons (vgl. 1. Stufe) in 1,5 l Tetrahydrofuran werden bei 0° C portionsweise mit 240 g (2,308 Mol) Formamidinacetat in 1,5 l Tetrahydrofuran versetzt. Man läßt das Reaktionsgemisch 15 Minuten bei 0° C nachrühren und versetzt es anschließend mit 298 g (2,5 Mol) N,N-Dimethylformamid-dimethylacetal. Man läßt das Reaktionsgemisch 1 Stunde bei 0° C und sechs Stunden bei 50-55° C nachrühren. Zur Aufarbeitung wird abgekühlt und vom Natrium-Acetat

abgesaugt. Das Filtrat wird fraktioniert destilliert. Man erhält 91,6 g (33,2 % der Theorie) Isopropyl-5-pyrimidinyl-keton vom Siedepunkt $Kp_{18}$ = 112-115 °C.

In analoger Weise und gemäß den angegebenen Verfahrensbedingungen können die folgenden 5-Acylpyrimidine der Formel (I)

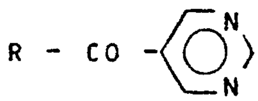

(I)

erhalten werden:

| Beispiel Nr. | R | Physikalische Konstante |
|---|---|---|
| 4 | 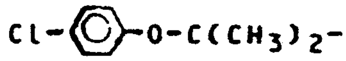 | Fp: 106 °C |
| 5 | 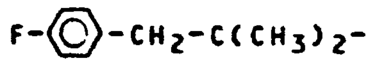 | $n_D^{20}$ = 1,5473 |
| 6 | 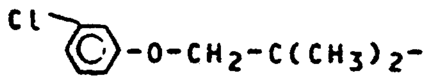 | Fp: 178 °C |
| 7 | 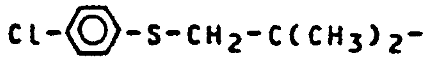 | Fp: 58 °C |
| 8 | $CH_3OCH_2-C(CH_3)_2-$ | $n_D^{20}$ = 1,4964 |
| 9 | 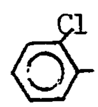 | Fp: 62 °C |
| 10 | 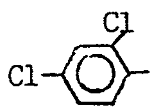 | Fp: 64 °C |
| 11 | 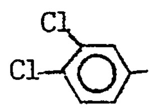 | Fp: 95 °C |
| 12 | 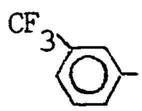 | Fp: 90-92 °C |

Herstellung von Benzyl-pyrimidinyl-alkylethern der Formel (X)

Beispiel (X-1)

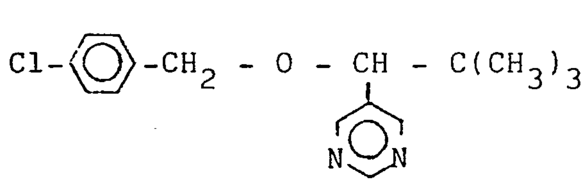

(X -1)

Herstellung des Ausgangsproduktes

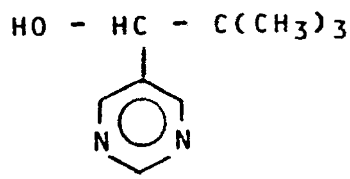

(XI-1)

16,4 g (0,1 Mol) tert.-Butyl-5-pyrimidinyl-keton in 100 ml Methanol werden bei Raumtemperatur mit 1,27 g (0,034 Mol) Natriumborhydrid in 10 ml Wasser versetzt. Man läßt das Reaktionsgemisch 1 Stunde bei Raumtemperatur rühren, engt im Vakuum ein, löst den Rückstand in Methylenchlorid und engt erneut im Vakuum ein. Man erhält 15,8 g (95 % der Theorie) 5-(1-Hydroxy-2,2-dimethyl-propyl)-pyrimidin vom Schmelzpunkt 93-95° C.

Herstellung des Endproduktes

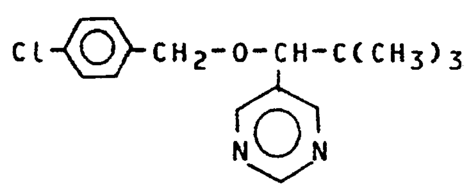

(X-1)

Eine Lösung von 16,6 g 5-(1-Hydroxy-2,2-dimethyl-propyl)-pyrimidin, 32,2 g 4-Chlorbenzylchlorid und 6 g Tetrabutylammoniumbromid in 200 ml Toluol wird mit 200 ml 33 %iger wäßriger Natronlauge versetzt. Das Reaktionsgemisch wird 18 Stunden bei Raumtemperatur kräftig gerührt.

Die wäßrige Phase wird abgetrennt, die organische Phase mit Toluol verdünnt, viermal mit Wasser, einmal mit gesättigter wäßriger Kochsalzlösung gewaschen, über Natriumsulfat getrocknet und eingeengt. Der ölige Rückstand wird in Ether/Hexan gelöst und mit Chlorwasserstoff begast. Der entstehende kristalline Niederschlag wird abgesaugt, mit Ether nachgewaschen und mit Essigester/1n wäßriger Natronlauge versetzt, wobei wieder die freie Base entsteht.

Nach Umkristallisation aus Hexan erhält man 20,3 g (70 % der Theorie) 5-[1-(4-Chlorbenzyloxy)-2,2-dimethylpropyl]-pyrimidin vom Schmelzpunkt 77-78,5° C.

Beispiel (X-2)

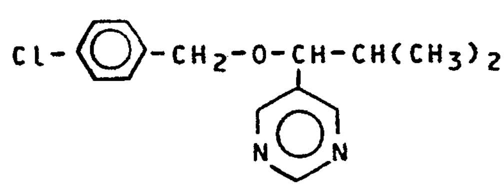

(X-2)

Herstellung des Ausgangsproduktes

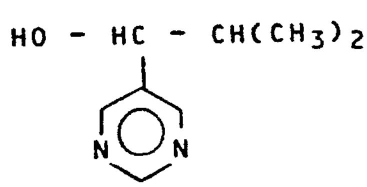

(XI-2)

15 g (0,1 Mol) Isopropyl-5-pyrimidinyl-keton in 100 ml Methanol werden bei Raumtemperatur mit 1,27 g (0,034 Mol) Natriumborhydrid in 10 ml Wasser versetzt. Man läßt das Reaktionsgemisch 1 Stunde bei Raumtemperatur nachrühren, engt im Vakuum ein, nimmt den Rückstand in Methylenchlorid auf und engt erneut im Vakuum ein. Man erhält 15,1 g (99,3 % der Theorie) 5-(1-Hydroxy-2-methyl-propyl)-pyrimidin vom Brechungsindex $n_D^{20}$ = 1,5082.

Herstellung des Endproduktes

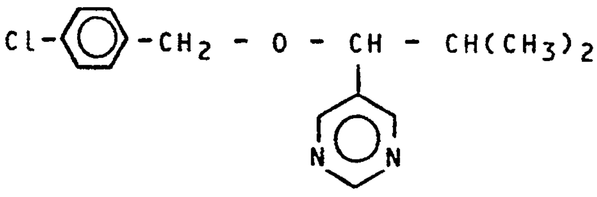

(X-2)

Eine Lösung von 6,8 g 5-(1-Hydroxy-2-methyl-propyl)-pyrimidin, 14,5 g 4-Chlorbenzylchlorid und 3 g Tetrabutylammoniumbromid in 100 ml Toluol wird mit 100 ml 33 %iger wäßriger Natronlauge versetzt. Das Reaktionsgemisch wird 2 Tage bei Raumtemperatur gerührt.

Die wäßrige Phase wird abgetrennt, die organische Phase mit Toluol verdünnt, viermal mit Wasser, einmal mit gesättigter wäßriger Kochsalzlösung gewaschen, über Natriumsulfat getrocknet und eingeengt. Der ölige Rückstand wird in Ether/Hexan gelöst und mit Chlorwasserstoff begast. Der entstehende kristalline Niederschlag abgesaugt, mit Ether nachgewaschen und mit Essigester/1n wäßriger Natronlauge versetzt, wobei wieder die freie Base entsteht.

Man erhält 10,2 g (82 % der Theorie) 5-[1-(4-Chlorbenzyloxy)-2-methylpropyl]-pyrimidin als helles Öl das langsam durchkristallisiert (Schmelzpunkt 33-35 °C).

Vergleichsbeispiel

Herstellung des 5-(1-Hydroxy-2,2-dimethyl-propyl)-pyrimidins der Formel (XI-1) nach dem bisher bekannten Verfahren:

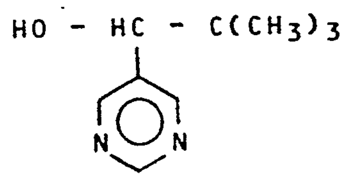

(XI-1)

225 g 5-Brompyrimidin werden in 1,5 l absolutem Tetrahydrofuran/1000 ml absolutem Ether gelöst, und die Lösung wird auf -120 °C abgekühlt. Bei einer Innentemperatur von -105 bis -115 °C werden 250 ml 50 %iges n-Butyllithium (in n-Hexan) innerhalb von 2 Stunden zugetropft. Es wird bei dieser Temperatur 1 Stunde nachgerührt. Dann werden 309 ml Trimethylacetaldehyd innerhalb von 2 Stunden zugetropft. Das Reaktionsgemisch wird anschließend 4 Stunden bei dieser Temperatur nachgerührt. Über Nacht läßt man das Reaktionsgemisch auf Raumtemperatur erwärmen und versetzt es anschließend mit 83 g Ammoniumchlorid, gelöst in einer minimalen Menge Wasser. Die wäßrige Phase wird abgetrennt, die organische Phase zweimal mit gesättigter wäßriger Kochsalzlösung gewaschen, über Natriumsulfat getrocknet und eingeengt. Nach Umkristallisation des Rückstandes aus Acetonitril erhält man 155 g (66 % der Theorie) 5-(1-Hydroxy-2,2-dimethylpropyl)-pyrimidin vom Schmelzpunkt 94-96 °C.

## Patentansprüche

1. Verfahren zur Herstellung von 5-Acylpyrimidinen der Formel

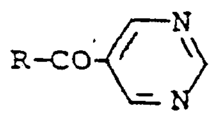

(I)

in welcher

R für Isopropyl, tert.-Butyl, n-Pentyl, Neopentyl, n-Hexyl, n-Heptyl, n-Octyl, geradkettiges oder verzweigtes Alkenyl mit 3 bis 6 Kohlenstoffatomen, geradkettiges oder verzweigtes Alkinyl mit 3 bis 6 Kohlenstoffatomen, für jeweils gegebenenfalls einfach bis dreifach gleich oder verschieden durch Alkyl mit 1 bis 4 Kohlenstoffatmomen substituiertes Cycloalkyl und Cycloalkylalkyl mit jeweils 3 bis 7 Kohlenstoffatomen im Cycloalkylteil und 1 bis 4 Kohlenstoffatomen im Alkylteil; für gegebenenfalls einfach bis dreifach, gleich oder verschieden substituiertes Phenyl, wobei als Substituenten genannt seien: Halogen; Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 5 gleichen oder verschiedenen Halogenatomen; Nitro, Cyano, Alkoxycarbonyl mit 1 bis 4 Kohlenstoffatomen im Alkoxyteil; sowie jeweils gegebenenfalls durch Halogen substituiertes Phenyl und Phenoxy; ferner für gegebenenfalls einfach bis dreifach, gleich oder verschieden substituiertes 5- bis 6-gliedriges Heteroaryl mit 1 bis 3 Sauerstoff-, Stickstoff-und/oder Schwefel, wobei als Substituenten die obengenannten Phenylsubstituenten infrage kommen, sowie für die Gruppierungen

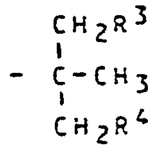 , 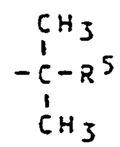 und 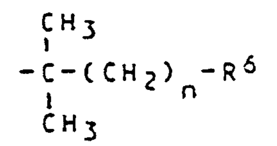 ,

wobei

$R^3$ für Fluor, Chlor oder Brom steht;

$R^4$ für Wasserstoff, Fluor, Chlor oder Brom steht;

$R^5$ für geradkettiges oder verzweigtes Alkenyl mit 2 bis 4 Kohlenstoffatomen, geradkettiges oder verzweigtes Alkinyl mit 3 bis 5 Kohlenstoffatomen, sowie für die CHO-Gruppe und Alkoximinomethyl mit 1 bis 4 Kohlenstoffatomen in jedem Alkoxyteil, Dialkoxymethyl mit 1 bis 4 Kohlenstoffatomen in jedem Alkoxyteil sowie gegebenenfalls substituierte Dioxolane und Dioxane steht;

$R^6$ für Cyano, für gegebenenfalls einfach bis dreifach, gleich oder verschieden substituiertes Phenyl steht, wobei als Substituenten die oben bereits genannten Phenylsubstituenten infrage kommen, sowie für die Gruppierungen $-XR^7$ und $-CONR^8R^9$ steht, wobei

$R^7$ für geradkettiges oder verzweigtes Alkyl mit 1 bis 6 Kohlenstoffatomen, Halogenalkyl mit 1 bis 4 Kohlenstoffatomen und 1 bis 5 gleichen. oder verschiedenen Halogenatomen sowie für jeweils gegebenenfalls einfach bis dreifach, gleich oder verschieden substituiertes Phenyl und Phenylalkyl mit 1 bis 2 Kohlenstoffatomen im Alkylteil steht, wobei jeweils als Substituenten die oben bereits genannten Phenylsubstituenten infrage kommen;

$R^8$ für Wasserstoff, geradkettiges oder verzweigtes Alkyl mit 1 bis 4 Kohlenstoffatomen oder für gegebenenfalls einfach bis dreifach, gleich oder verschieden substituiertes Phenyl steht, wobei als Substituenten die oben bereits genannten Phenylsubstituenten infrage kommen;

$R^9$ für Wasserstoff oder geradkettiges oder verzweigtes Alkyl mit 1 bis 4 Kohlenstoffatomen steht;

X für Sauerstoff oder Schwefel steht und

n für die Zahlen 0, 1 oder 2 steht,

dadurch gekennzeichnet, daß man Methylketone der Formel

$R - CO - CH_3$ (II)

in welcher

R die oben angegebene Bedeutung hat,

a) in einer 1. Stufe mit einem Ameisensäurealkylester mit 1 bis 4 Kohlenstoffatomen in der Alkylgruppe oder Ameisensäure-phenylester in Gegenwart und von Alkalialkoholaten gegebenenfalls in Gegenwart eines Verdünnungsmittels umsetzt und gegebenenfalls ansäuert; danach in einer 2. Stufe die entstehenden Enol-ketone der Formel

$R\text{-}CO\text{-}CH=CH\text{-}OR^{10}$ (III)

in welcher
R die oben angegebene Bedeutung hat und
$R^{10}$ für Wasserstoff oder einen Baserest steht,
mit oder ohne Zwischenisolierung mit Formamidin, wobei das Formamidin gegebenenfalls in Form eines Formamidinsalzes vorliegen kann, gegebenenfalls in Gegenwart eines Verdünnungsmittels, umsetzt;

anschließend in einer 3. Stufe die entstehenden Formamidin-Derivate der Formel

$R\text{-}CO\text{-}CH_2\text{-}CH=N\text{-}CH=NH$ (IV)

in welcher
R die oben angegebene Bedeutung hat,
mit oder ohne Zwischenisolierung mit einem
α) Formamid-Acetale der Formel

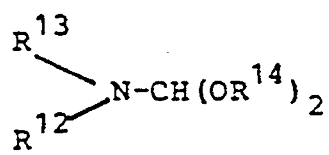

(VII)

in welcher
$R^{12}$ und $R^{13}$ unabhängig voneinander für Alkyl mit 1 bis 4 Kohlenstoffatomen stehen und
$R^{14}$ für Alkyl mit 1 bis 4 Kohlenstoffatomen steht,
oder
β) Aminalester der Formel

$R^{14}O\text{-}CH(NR^{12}R^{13})_2$ (VIII)

in welcher
$R^{12}$, $R^{13}$ und $R^{14}$ die oben angegebene Bedeutung haben;
oder
γ) Formiminium-Halogenide der Formel

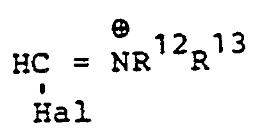

(IX)

in welcher
$R^{12}$ und $R^{13}$ die oben angegebene Bedeutung haben und
Hal für Halogen steht,
gegebenenfalls in Gegenwart eines Verdünnungsmittels, bei Temperaturen zwischen - 10 und + 10 °C umsetzt;
und in einer 4. Stufe die entstehenden Verbindungen der Formel

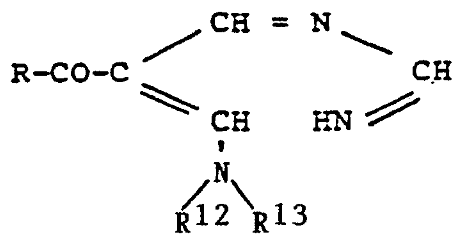

(V)

in welcher

R die oben angegebene Bedeutung hat und

mit oder ohne Zwischenisolierung thermisch cyclisiert.

2. 5-Acyl-pyrimidine der Formel

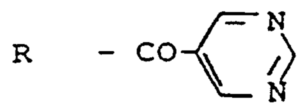

(I)

in welcher

R die in Anspruch 1 für R angegebene Bedeutung hat.

3. Verwendung von 5-Acyl-pyrimidinen der Formel (I) gemäß Anspruch 2 als Zwischenprodukte zur Herstellung von Benzylpyrimidinyl-alkylethern.

**Claims**

1. Process for the preparation of 5-acylpyrimidines of the formula

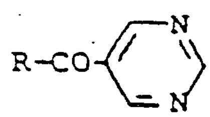

(I)

in which

R represents isopropyl, tert.-butyl, n-pentyl, neopentyl, n-hexyl, n-heptyl, n-octyl, straight-chain or branched alkenyl with 3 to 6 carbon atoms, straight-chain or branched alkinyl with 3 to 6 carbon atoms or cycloalkyl and cycloalkylalkyl with in each case 3 to 7 carbon atoms in the cycloalkyl moiety and 1 to 4 carbon atoms in the alkyl moiety and in each case optionally mono-, di- or tri-substituted by identical or different alkyl substituents with 1 to 4 carbon atoms; or represents phenyl which is optionally mono-, di- or tri-substituted by identical or different substituents, substituents which may be mentioned being: halogen; alkyl, alkoxy and alkylthio with in each case 1 to 4 carbon atoms; halogenoalkyl, halogenoalkoxy and halogenoalkylthio with in each case 1 to 4 carbon atoms and 1 to 5 identical or different halogen atoms; nitro, cyano and alkoxycarbonyl with 1 to 4 carbon atoms in the alkoxy moiety; and phenyl and phenoxy, in each case optionally substituted by halogen; or furthermore represents 5- to 6-membered heteroaryl which has 1 to 3 oxygen, nitrogen and/or sulphur atoms, and is optionally mono-, di- or tri-substituted by identical or different substituents, possible substituents being the abovementioned substituents on phenyl, or represents the groupings

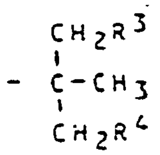, 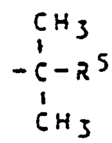 and 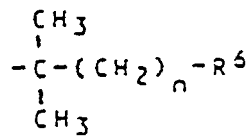,

where

$R^3$ represents fluorine, chlorine or bromine;

$R^4$ represents hydrogen, fluorine, chlorine or bromine;

$R^5$ represents straight-chain or branched alkenyl with 2 to 4 carbon atoms, straight-chain or branched alkinyl with 3 to 5 carbon atoms, or the CHO group and alkoximinomethyl with 1 to 4 carbon atoms in each alkoxy moiety, dialkoxymethyl with 1 to 4 carbon atoms in each alkoxy moiety and optionally substituted dioxolanes and dioxanes;

$R^6$ represents cyano, or represents phenyl which is optionally mono-, di- or tri-substituted by identical or different substituents, possible substituents being the substituents on phenyl which have already been mentioned above, or represents the groupings $-XR^7$ and $-CONR^8R^9$, wherein

$R^7$ represents straight-chain or branched alkyl with 1 to 6 carbon atoms or halogenoalkyl with 1 to 4 carbon atoms and 1 to 5 identical or different halogen atoms, or represents phenyl or phenylalkyl, with 1 or 2 carbon atoms in the alkyl moiety, each of which is optionally mono-, di- or tri-substituted by identical or different substituents, possible substituents in each case being the substituents on phenyl which have already been mentioned above;

$R^8$ represents hydrogen or straight-chain or branched alkyl with 1 to 4 carbon atoms, or represents phenyl which is optionally mono-, di- or tri-substituted by identical or different substituents, possible substituents being the substituents on phenyl which have already been mentioned above;

$R^9$ represents hydrogen or straight-chain or branched alkyl with 1 to 4 carbon atoms;

X represents oxygen or sulphur and

n represents the number 0, 1 or 2,

characterised in that methyl ketones of the formula

$R-CO-CH_3$ (II)

in which

R has the abovementioned meaning,

a) are reacted in a 1st stage with an alkyl formate with 1 to 4 carbon atoms in the alkyl group or phenyl formate in the presence of alkali metal alcoholates and if appropriate in the presence of a diluent and, if appropriate, the product is acidified; thereafter, in a 2nd stage, the enol ketones formed, of the formula

$R-CO-CH=CH-OR^{10}$ (III)

in which

R has the abovementioned meaning and

$R^{10}$ represents hydrogen or a base radical, are reacted, with or without intermediate isolation, with formamidine, it being possible for the formamidine optionally to be in the form of a formamidine salt, if appropriate in the presence of a diluent;

and subsequently, in a 3rd stage, the formamidine derivatives formed, of the formula

$R-CO-CH_2-CH=N-CH=NH$ (IV)

in which

R has the abovementioned meaning, are reacted, with or without intermediate isolation, with a

α) formamide acetal of the formula

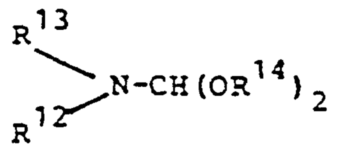

(VII)

in which

$R^{12}$ and $R^{13}$ independently of one another represent alkyl with 1 to 4 carbon atoms and

$R^{14}$ represents alkyl with 1 to 4 carbon atoms,

or

β) aminal ester of the formula

$R^{14}$-O-CH($NR^{12}R^{13}$)$_2$ (VIII)

in which

$R^{12}$, $R^{13}$ and $R^{14}$ have the abovementioned meaning;

or

γ) formiminium halide of the formula

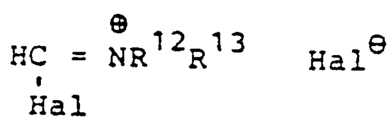

(IX)

in which

$R^{12}$ and $R^{13}$ have the abovementioned meaning and

Hal represents halogen, if appropriate in the presence of a diluent, at temperatures between -10 and +10° C;

and, in a 4th stage, the compounds formed, of the formula

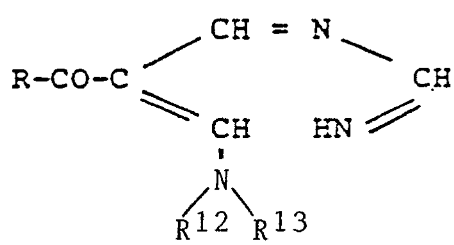

(V)

in which

R has the abovementioned meaning,

are cyclised by means of heat, with or without intermediate isolation.

**2.** 5-Acyl-pyrimidineS of the formula

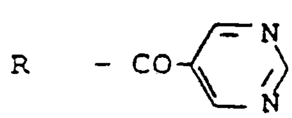

(I)

in which

R has the meaning specified for R in Claim 1.

**3.** Use of 5-acyl-pyrimidines of the formula (I) according to Claim 2 as intermediates for the preparation of benzylpyrimidinyl alkyl ethers.

## Revendications

**1.** Procédé de préparation de 5-acylpyrimidines de formule

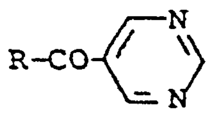

(I)

dans laquelle

R représente un groupe isopropyle, tertio-butyle, n-pentyle, néopentyle, n-hexyle, n-heptyle, n-octyle, un groupe alcényle à chaîne droite ou ramifiée ayant 3 à 6 atomes de carbone, un groupe alcynyle à chaîne droite ou ramifiée ayant 3 à 6 atomes de carbone, un groupe cycloalkyle et un groupe cycloalkylalkyle ayant chacun 3 à 7 atomes de carbone dans la partie cycloalkyle et 1 à 4 atomes de carbone dans la partie alkyle, portant chacun le cas échéant un à trois substituants alkyle en $C_1$ à $C_4$ identiques ou différents ; un groupe phényle portant éventuellement un à trois substituants identiques ou différents, parmi lesquels on mentionne : un halogène ; un radical alkyle, alkoxy et alkylthio ayant chacun 1 à 4 atomes de carbone ; un radical halogénalkyle, halogénalkoxy et halogénalkylthio ayant chacun 1 à 4 atomes de carbone et 1 à 5 atomes d'halogènes identiques ou différents ; un radical nitro, cyano, alkoxycarbonyle ayant 1 à 4 atomes de carbone dans la partie alkoxy ; ainsi qu'un groupe phényle et un groupe phénoxy portant chacun le cas échéant un substituant halogéno ; en outre, un groupe hétéroaryle pentagonal ou hexagonal ayant 1 à 3 atomes d'oxygène, d'azote et/ou de soufre, portant le cas échéant un à trois substituants identiques ou différents, en considérant comme substituants les substituants du groupe phényle mentionnés ci-dessus, ainsi que les groupements

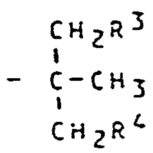 , 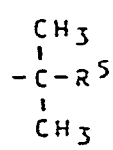 et 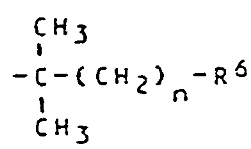

où

$R^3$ représente le fluor, le chlore ou le brome ;

$R^4$ est l'hydrogène, le fluor, le chlore ou le brome ;

$R^5$ est un groupe alcényle à chaîne droite ou ramifiée ayant 2 à 4 atomes de carbone, un groupe alcynyle à chaîne droite ou ramifiée ayant 3 à 5 atomes de carbone, ainsi que le groupe CHO et un groupe alkoxyiminométhyle ayant 1 à 4 atomes de carbone dans chaque partie alkoxy, un groupe dialkoxyméthyle ayant 1 à 4 atomes de carbone dans chaque partie alkoxy ainsi que des dioxolannes et des dioxannes éventuellement substitués,

$R^6$ est un groupe cyano, un groupe phényle portant éventuellement un à trois substituants identiques ou différents, en considérant comme substituants les substituants du groupe phényle déjà mentionnés ci-dessus, ainsi que les groupements $-XR^7$ et $-CONR^8R^9$, où

$R^7$ représente un groupe alkyle à chaîne droite ou ramifiée ayant 1 à 6 atomes de carbone, un groupe halogénalkyle ayant 1 à 4 atomes de carbone et 1 à 5 atomes d'halogènes identiques ou différents, ainsi qu'un groupe phényle et un groupe phénylalkyle ayant 1 ou 2 atomes de carbone dans la partie alkyle, portant chacun le cas échéant un à trois substituants identiques ou différents, en considérant dans chaque cas comme substituants les substituants du groupe phényle déjà mentionnés ci-dessus ;

$R^8$ représente l'hydrogène, un groupe alkyle à chaîne droite ou ramifiée ayant 1 à 4 atomes de carbone ou un groupe phényle portant éventuellement un à trois substituants identiques ou différents, en considérant comme substituants les substituants du groupe phényle déjà mentionnés ci-dessus ;

$R^9$ représente l'hydrogène ou un groupe alkyle à chaîne droite ou ramifiée ayant 1 à 4 atomes de carbone ;

X représente l'oxygène ou le soufre et
n a les valeurs 0, 1 ou 2,
caractérisé en ce qu'on fait réagir des méthylcétones de formule

$R - CO - CH_3$ (II)

dans laquelle
R a la définition indiquée ci-dessus,
a) dans une première étape avec un ester alkylique d'acide formique ayant 1 à 4 atomes de carbone dans le groupe alkyle ou avec l'ester de phényle de l'acide formique,
en présence d'alcoolates alcalins et le cas échéant en présence d'un diluant, et en acidifiant le cas échéant ; après quoi, dans une deuxième étape, on fait réagir les énol-cétones produites de formule

$R\text{-}CO\text{-}CH = CH\text{-}OR^{10}$ (III)

dans laquelle
R a la définition indiquée ci-dessus et
$R^{10}$ représente l'hydrogène ou un reste de base,
avec ou sans isolement intermédiaire, avec la formamidine, cette dernière pouvant être présente le cas échéant sous forme d'un sel de formamidine, éventuellement en présence d'un diluant ;
puis, dans une troisième étape, on fait réagir les dérivés de formamidine produits de formule

$R\text{-}CO\text{-}CH_2\text{-}CH = N\text{-}CH = NH$ (IV)

dans laquelle
R a la définition indiquée ci-dessus, avec ou sans isolement intermédiaire, avec
α) un formamido-acétal de formule

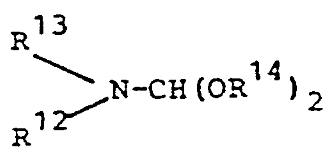

(VII)

dans laquelle
$R^{12}$ et $R^{13}$ représentent indépendamment l'un de l'autre un groupe alkyle ayant 1 à 4 atomes de carbone et
$R^{14}$ est un groupe alkyle ayant 1 à 4 atomes de carbone,
ou bien
β) un aminal-ester de formule

$R^{14}O\text{-}CH(NR^{12}R^{13})_2$ (VIII)

dans laquelle
$R^{12}$, $R^{13}$ et $R^{14}$ ont la définition indiquée ci-dessus ;
ou bien
γ) des halogénures de formiminium de formule

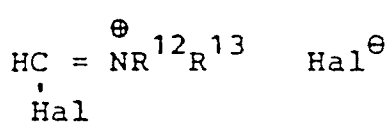

(IX)

dans laquelle
$R^{12}$ et $R^{13}$ ont la définition indiquée ci-dessus et
Hal représente un halogène,

le cas échéant en présence d'un diluant ; à des températures comprises entre -10 et +10 °C ; et dans une quatrième étape, on cyclise thermiquement les composés produits de formule

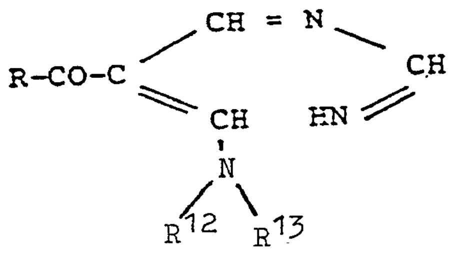

(V)

dans laquelle

R a la définition indiquée ci-dessus avec ou sans isolement intermédiaire.

**2.** 5-acylpyrimidines de formule

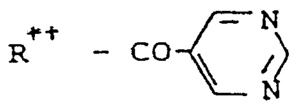

(I)

dans laquelle

$R^{++}$ a la définition indiquée pour R dans la revendication 1.

**3.** Utilisation de 5-acylpyrimidines de formule (I) suivant la revendication 2 comme produits intermédiaires pour l'obtention d'éthers de benzylpyrimidinyle et d'alkyle.